(19) Europäisches Patentamt
European Patent Office
Office européen des brevets

(11) **EP 4 692 306 A1**

(12) **DEMANDE DE BREVET EUROPEEN**

(43) Date de publication:
**11.02.2026 Bulletin 2026/07**

(21) Numéro de dépôt: **25184390.0**

(22) Date de dépôt: **23.06.2025**

(51) Classification Internationale des Brevets (IPC):
*C12M 3/06* (2006.01)          *B01L 3/00* (2006.01)
*C12M 1/34* (2006.01)

(52) Classification Coopérative des Brevets (CPC):
**C12M 23/16; B01L 3/5027; B01L 3/502715;**
**C12M 41/46;** B01L 2300/0645; B01L 2300/0816

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **09.08.2024 FR 2408830**

(71) Demandeur: **Commissariat à l'Energie Atomique et aux Energies Alternatives**
**75015 Paris (FR)**

(72) Inventeurs:
• **THOMAS, Yohann**
  **38054 Grenoble Cedex 09 (FR)**
• **DEN DULK, Remco**
  **38054 Grenoble Cedex 09 (FR)**

(74) Mandataire: **INNOV-GROUP**
   **209 Avenue Berthelot**
   **69007 Lyon (FR)**

(54) **PROCÉDÉ ET SYSTÈME DE SURVEILLANCE D'UN OBJET BIOLOGIQUE**

(57) L'invention concerne un procédé de surveillance d'un objet biologique (O) placé dans un composant microfluidique (OOC), mis en œuvre à l'aide d'un système de surveillance qui comporte un capteur (SENS), le procédé de surveillance comportant :
- Une étape de calibration du capteur (SENS),
- Une première étape de perfusion de l'objet biologique (O) pour générer à l'aide du capteur des données de mesure de référence,
- Une deuxième étape de perfusion de l'objet biologique par injection d'une première solution de perfusion (P1) dans le composant microfluidique (OOC), ladite première solution de perfusion (P1) prenant un deuxième état après avoir traversé l'objet biologique (O),
- Ladite première solution de perfusion (P1) dans son deuxième état étant injectée dans ledit capteur (SENS) pour générer à l'aide du capteur des deuxièmes données de mesure,
- Une étape de mesure différentielle par le capteur (SENS), par comparaison des données de mesure de référence avec les deuxièmes données de mesure.

Fig. 1

## Description

## Domaine technique de l'invention

La présente invention se rapporte à un procédé et un système de surveillance d'un objet biologique.

## Etat de la technique

Pour simuler la fonctionnalité d'organes humains, il est désormais connu d'utiliser un composant microfluidique dans lequel on vient placer des cellules humaines à étudier, ce composant étant dénommé couramment organe sur puce ou « organ on chip » en anglais.

L'objectif est de suivre en temps réel le comportement d'un objet biologique ainsi que d'évaluer l'effet des médicaments ou des contraintes physiques directement sur les cellules qui composent l'objet biologique.

Pour suivre l'évolution de certains paramètres lors de la surveillance de l'objet biologique, on utilise un ou plusieurs capteurs électrochimiques (ampérométrique ou potentiométrique par exemple), associés à l'organe sur puce.

Il est connu de l'état de l'art que ces capteurs électrochimiques présentent une dérive intrinsèque de leur réponse électrochimique (quel que soit le type de mode de transduction du capteur). Cette dérive a un impact direct sur la précision et la fiabilité des mesures. Par ailleurs, la réponse électrochimique d'un capteur peut varier selon le milieu utilisé. Sa réponse sera notamment plus précise pour des solutions « simples » (typiquement une ou des solutions de calibration) que pour des solutions complexifiées telles que celles utilisées lors des études d'objets biologiques (solutions de perfusion contenant une plus grande variété de constituants potentiellement impactant la réponse du capteur).

Lorsque l'expérimentation sur un objet biologique est particulièrement longue, il peut s'avérer nécessaire de calibrer régulièrement les capteurs électrochimiques employés, ainsi que de procéder à des étalonnages réguliers.

Pour rappel, la calibration d'un capteur consiste à venir déterminer à la fois sa sensibilité et son décalage (appelé couramment « offset »). Pour cela, on vient souvent utiliser plusieurs solutions de calibration (au moins deux solutions de calibration).

L'étalonnage d'un capteur s'effectue classiquement avec une solution dite étalon et permet de comparer la valeur mesurée par le capteur (par exemple une concentration) avec une référence ou étalon. L'étalonnage permet de venir régler le décalage du capteur.

La demande de brevet WO2013086486A1 décrit des systèmes microphysiologiques intégrés d'organes sur puce représentant des organes vivants et des structures de support pour ces systèmes. Les systèmes incorporent plusieurs capteurs (électrochimiques), plusieurs solutions de nutritions, de calibration et de nettoyage. Les calibrations des capteurs sont réalisées de manière périodique. Les systèmes permettent l'étude de plusieurs type d'objets biologiques. Cette demande de brevet ne prévoit pas de solutions simples pour étalonner les capteurs utilisés. Or, il est nécessaire de prévoir des étalonnages et des calibrations de manière régulière, notamment lorsque l'expérimentation s'avère particulièrement longue et que chaque capteur est susceptible de dériver avec le temps.

La demande de brevet WO2021/054280A1 et le brevet US9034572 concernent également une méthode d'étalonnage de capteurs.

Le but de l'invention est de proposer une solution technique permettant, dans le cadre de la surveillance d'un objet biologique, de réaliser à la fois des étalonnages et des calibrations du capteur électrochimique, sans interruption de l'expérimentation sur l'objet biologique.

## Exposé de l'invention

Ce but est atteint par un procédé de surveillance d'un objet biologique placé dans un composant microfluidique, ledit composant microfluidique comportant une première entrée microfluidique et une première sortie microfluidique, mis en œuvre à l'aide d'un système de surveillance qui comporte un capteur, ledit capteur comportant une deuxième entrée microfluidique et une deuxième sortie microfluidique, le procédé de surveillance comportant :

- Une première étape d'injection d'une première solution de calibration, via un circuit microfluidique de calibration, à travers le capteur, par la deuxième entrée microfluidique,
- Une deuxième étape d'injection d'une deuxième solution de calibration, via le circuit microfluidique de calibration, à travers le capteur, par la deuxième entrée microfluidique,
- Une première étape de calibration du capteur après ladite première étape d'injection et ladite deuxième étape d'injection,
- Une première étape de perfusion de l'objet biologique par injection dans un circuit microfluidique de perfusion d'une première solution de perfusion dans le composant microfluidique, via la première entrée microfluidique, ladite première solution de perfusion étant injectée également dans un premier état dit de référence, en parallèle dans le capteur électrochimique (SENS) via la deuxième entrée microfluidique pour générer à l'aide du capteur des données de mesure de référence,
- Une deuxième étape de perfusion de l'objet biologique par injection dans le circuit microfluidique de perfusion de la première solution de perfusion dans le composant microfluidique, via la première entrée microfluidique, ladite première solution de perfusion prenant un deuxième état après avoir traversé l'objet biologique,

- Ladite première solution de perfusion dans son deuxième état étant injectée dans ledit capteur via une liaison fluidique reliant la première sortie microfluidique à la deuxième entrée microfluidique, pour générer à l'aide du capteur des deuxièmes données de mesure,
- Une étape de mesure différentielle par le capteur, par comparaison des données de mesure de référence avec les deuxièmes données de mesure.

Selon une particularité, le procédé comporte une première étape de lavage du circuit microfluidique de calibration, mise en œuvre après la première étape de calibration.

Selon une autre particularité, la première étape d'injection et la deuxième étape d'injection sont mises en œuvre pour déterminer la sensibilité et le décalage du capteur. Selon une autre particularité, le procédé consiste à répéter une ou plusieurs fois la première étape de perfusion en vue de surveiller la première solution de perfusion. Selon une autre particularité, le procédé consiste à faire suivre chaque itération de la première étape de perfusion par une itération de la deuxième étape de perfusion, en vue de mettre en œuvre une nouvelle étape de mesure différentielle et de suivre l'évolution de la mesure différentielle au cours du temps.

L'invention concerne également un système de surveillance d'un objet biologique placé dans un composant microfluidique, ledit composant microfluidique comportant une première entrée microfluidique et une première sortie microfluidique et étant placé dans un système microfluidique, le système microfluidique comportant également :

- Un capteur ayant une deuxième entrée microfluidique et une deuxième sortie microfluidique,
- Un circuit microfluidique de calibration connecté sur la deuxième entrée microfluidique du capteur électrochimique, ledit circuit microfluidique de calibration comportant au moins un premier réservoir d'une première solution de calibration et un deuxième réservoir d'une deuxième solution de calibration,
- Un circuit microfluidique de perfusion connecté sur la première entrée microfluidique du composant microfluidique et sur la deuxième entrée microfluidique du capteur électrochimique, le circuit microfluidique de perfusion comportant au moins un réservoir d'une première solution de perfusion,
- Une unité de commande configurée pour contrôler le circuit microfluidique de perfusion et le circuit microfluidique de calibration,
- Ladite première sortie microfluidique du composant microfluidique étant reliée par une liaison microfluidique à ladite deuxième entrée microfluidique du capteur,
- L'unité de commande étant configurée pour contrôler le circuit microfluidique de perfusion et le circuit microfluidique de calibration en vue de mettre en œuvre les étapes du procédé de surveillance de l'objet biologique tel que défini ci-dessus.

Selon une particularité, le système comporte un circuit microfluidique de lavage ayant un réservoir d'une solution de lavage, connecté à la fois à la première entrée microfluidique du composant microfluidique et à la deuxième entrée microfluidique du capteur.

Selon une autre particularité, le circuit microfluidique de calibration comporte un troisième réservoir d'une troisième solution de calibration.

Selon une autre particularité, le capteur est de type électrochimique.

La solution de l'invention utilise une ou plusieurs solutions de perfusion ainsi que au moins deux solutions de calibration. Grâce à l'invention, il est possible de réaliser une calibration et/ou un étalonnage du capteur à la demande, et donc de prendre en compte la dérive du capteur en temps réel. Par ailleurs, il sera également possible de corriger les mesures effectuées par le capteur, en temps réel ou a posteriori, en tenant compte de la dérive du capteur calculée.

Il est ainsi possible de surveiller l'objet biologique en temps réel, sur des expérimentations longues, sans interruption, tout en conservant des mesures électrochimiques fiables et exploitables au cours du temps.

**Brève description des figures**

D'autres caractéristiques et avantages vont apparaître dans la description détaillée qui suit faite en regard des dessins annexés dans lesquels :

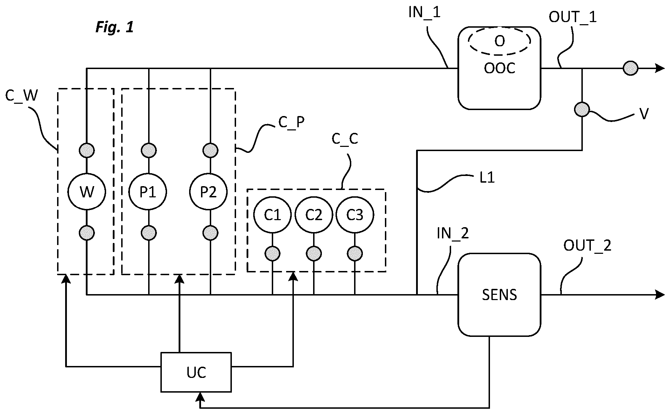

- La figure 1 représente de manière schématique l'architecture du système de surveillance conforme à l'invention ;
- Les figures 2A à 2J représentent les différentes étapes du procédé de l'invention ;
- La figure 3 montre un diagramme temporel illustrant le principe de fonctionnement de l'invention ;

**Description détaillée d'au moins un mode de réalisation**

L'invention concerne un système de surveillance d'un objet biologique. L'objet biologique à étudier est placé dans un composant microfluidique OOC, appelé également « Organ On Chip ».

L'objet biologique O est par exemple un agrégat de cellules. Par agrégat de cellules, on entend, selon l'invention, l'auto-assemblage d'un ou plusieurs types de cellules en deux ou trois dimensions. Un tel agrégat de cellules peut notamment s'appeler sphéroïde, organoïde, tumoroïde, neuro-sphère. Cet agrégat peut également être un îlot de Langerhans ou un modèle de peau.

Dans la suite de la description, on utilisera de manière générique le terme "objet biologique", référencé O, pour évoquer un tel agrégat, ce terme étant classiquement employé dans le domaine de la culture de cellules vivantes. De manière non limitative, un tel objet biologique O peut par exemple présenter un diamètre allant de quelques dizaines de μm à quelques centaines de μm.

Le composant microfluidique OOC comporte une première entrée microfluidique IN_1, située en amont de l'objet biologique O et par laquelle il peut recevoir notamment une solution de perfusion microfluidique à destination de l'objet biologique, et une première sortie microfluidique OUT_1, positionnée en aval par rapport à l'objet biologique O, et de laquelle la solution de perfusion est évacuée après avoir été en contact avec l'objet biologique présent dans le composant mcirofluidique.

En plus du composant microfluidique OOC, le système de surveillance comporte également :

- Au moins un capteur SENS ayant une deuxième entrée microfluidique IN_2 et une deuxième sortie microfluidique OUT_2,
- Un circuit microfluidique de perfusion C_P connecté sur la première entrée microfluidique IN_1 du composant microfluidique et sur la deuxième entrée microfluidique IN_2 du capteur SENS, le circuit microfluidique de perfusion comportant au moins un réservoir d'une première solution de perfusion P1,
- Un circuit microfluidique de calibration C_C connecté sur la deuxième entrée microfluidique IN_2 du capteur SENS, ledit circuit microfluidique de calibration C_C comportant au moins un premier réservoir d'une première solution de calibration C1 et un deuxième réservoir d'une deuxième solution de calibration C2,
- Une unité de commande UC configurée pour contrôler le circuit microfluidique de perfusion C_P et le circuit microfluidique de calibration C_C.

Le capteur SENS est avantageusement de type électrochimique, mais l'invention peut tout à fait s'appliquer à un autre type de capteur, de type optique par exemple.

Sur les figures annexées, le circuit microfluidique de calibration C_C comporte par exemple trois réservoirs de solutions de calibration C1, C2, C3, en vue de fiabiliser encore plus le système.

De même, le circuit microfluidique de perfusion C_P comporte deux réservoirs agencés en parallèle contenant chacun une solution de perfusion P1, P2 distincte.

Pour contrôler le circuit microfluidique de perfusion C_P et le circuit microfluidique de calibration C_C, l'unité de commande UC agit sur des vannes microfluidiques V et des moyens de pompage agencés dans les différents circuits.

Ainsi, une vanne distincte est positionnée en sortie de chaque réservoir du circuit microfluidique de calibration C_C.

Et deux vannes distinctes sont positionnées de part et d'autre de chaque réservoir du circuit microfluidique de perfusion C_P, une première vanne sur la liaison fluidique conduisant au composant microfluidique OOC et une deuxième vanne sur la liaison fluidique conduisant au capteur SENS.

Le système comporte également avantageusement au moins un circuit microfluidique de lavage C_W comportant au moins un réservoir contenant une solution de lavage W, le circuit microfluidique de lavage C_W comportant au moins une première liaison fluidique connectée à la première entrée microfluidique IN_1 du composant microfluidique OOC et une deuxième liaison fluidique connectée à la deuxième entrée microfluidique IN_2 du capteur SENS. Deux vannes distinctes sont positionnées sur ce circuit de lavage C_W, de part et d'autre de son réservoir contenant la solution de lavage W. L'injection de la solution de lavage dans le système est également contrôlée par l'unité de commande UC, via le contrôle des moyens de pompage et des vannes.

Les moyens de pompage (non représentés) sont agencés dans les différents circuits du système pour assurer une circulation des fluides :

- Du circuit microfluidique de calibration C_C vers le capteur SENS ;
- Du circuit microfluidique de perfusion C_P vers le composant microfluidique OOC contenant l'objet biologique O et/ou vers le capteur SENS ;
- Du circuit microfluidique de lavage C_W vers le composant microfluidique OOC et/ou vers le capteur SENS ;

Les moyens de pompage peuvent comporter plusieurs pompes de type péristaltique, à vide et/ou à pression, et/ou des moyens de pompage externes venant se connecter au circuit microfluidique.

Les vannes V employées sont avantageusement de type pneumatique.

Selon l'invention, le système comporte également une liaison fluidique L1 spécifique reliant la première sortie microfluidique OUT_1 du composant microfluidique OOC à la deuxième entrée microfluidique IN_2 du capteur SENS. Grâce à ce mécanisme, il est possible de réaliser un étalonnage du capteur SENS à l'aide d'une solution de perfusion dans un premier état, dit état de référence, avant passage dans le composant microfluidique, et la même solution de perfusion récupérée dans un deuxième état après son passage dans le composant microfluidique OOC, après perfusion de l'objet biologique.

Il faut noter que la solution de perfusion peut être aussi appelée solution de nutrition, milieu de culture...

Le schéma du système permet de calibrer insitu le capteur SENS, de suivre la possible évolution de chaque solution de perfusion P1, P2 en termes de composition (selon le type de capteur), et enfin de réaliser des étalonnages réguliers (à l'aide d'une solution de perfusion selon le principe général décrit ci-dessus).

Pour rappel, la calibration d'un capteur SENS consiste à établir une courbe de calibration (dont les deux paramètres expérimentaux sont la sensibilité, désignée $\alpha$, et le décalage désigné $\beta$ - appelé aussi offset) à partir d'au moins deux points de mesure (c'est-à-dire deux solutions de calibration distinctes). Et l'étalonnage d'un capteur s'effectue avec au moins un point de mesure en utilisant une solution étalon qui, par comparaison avec une autre solution (perfusion ou autre) permet de suivre le décalage du capteur.

A titre d'exemple, dans le cas d'un capteur électrochimique de type potentiométrique, la corrélation entre le potentiel mesuré et la concentration de l'analyte d'intérêt est donné par l'équation de Nernst :

$$E = \alpha \log C + \beta$$

En imaginant des concentrations $C_1$ et $C_2$ correspondant à des mesures de potentiel $E_1$ et $E_2$ au niveau du capteur, les équations suivantes peuvent être établies :

$$E_1 = \alpha \log C_1 + \beta$$

Et

$$E_2 = \alpha \log C_2 + \beta$$

Il est alors possible d'utiliser ces deux équations afin d'obtenir la relation suivante :

$$E_2 - E_1 = \alpha \log \frac{C_2}{C_1}$$

La valeur de la concentration $C_2$ est alors obtenue selon :

$$C_2 = C_1 \, 10^{(E_2 - E_1)/\alpha}$$

Une méthode de calcul similaire est également valable pour un capteur électrochimique de type ampérométrique. Cette fois-ci, la concentration d'un analyte est proportionnelle au courant mesuré, $i$ (suite à l'imposition d'un potentiel sur l'électrode) selon :

$$i = \alpha \, C + \beta$$

Par le même raisonnement que précédemment, nous obtenons pour deux concentrations, $C_1$ et $C_2$ :

$$i_1 = \alpha \, C_1 + \beta \text{ et } i_2 = \alpha \, C_2 + \beta$$

En réarrangeant ces deux équations, le calcul de la valeur de la concentration $C_2$ devient alors :

$$C_2 = C_1 + \frac{i_2 - i_1}{\alpha}$$

Les deux exemples présentés ci-dessus mettent en évidence qu'un étalonnage du capteur électrochimique (potentiométrique ou ampérométrique) permet de s'affranchir du décalage de mesure (offset $\beta$) qui, il est bien connu, évolue dans le temps et est largement dépendant des conditions externes des milieux (température, composition, taux d'oxygène, encrassement du capteur,...).

Dans le cadre de l'invention, on peut ainsi avancer les hypothèses/considérations ci-dessous sur la réponse d'un capteur :

- Décalage (β) :

  ◦ Il évolue au cours du temps, par dérive intrinsèque du capteur ;
  ◦ Il peut être impacté par la solution faisant l'objet de la mesure, d'où l'intérêt de mesurer consécutivement (étalonner) deux solutions de même nature afin de vérifier si le décalage reste identique ;

- Sensibilité (α) :

  ◦ Elle peut légèrement diminuer sur le long terme d'où l'intérêt de réaliser des courbes de calibration en début et fin d'expérimentation ;
  ◦ Elle peut être légèrement différente d'un milieu à un autre (milieu simple vers complexe par exemple), mais son impact reste assez négligeable sur une durée courte (étalonnage) ;

Dans ce dernier cas, il faut noter que la sensibilité pourra être impactée par exemple si des interférences ou bien si la sélectivité du capteur par rapport à une espèce secondaire est amoindrie. On peut cependant considérer que la sensibilité reste constante lors d'un étalonnage.

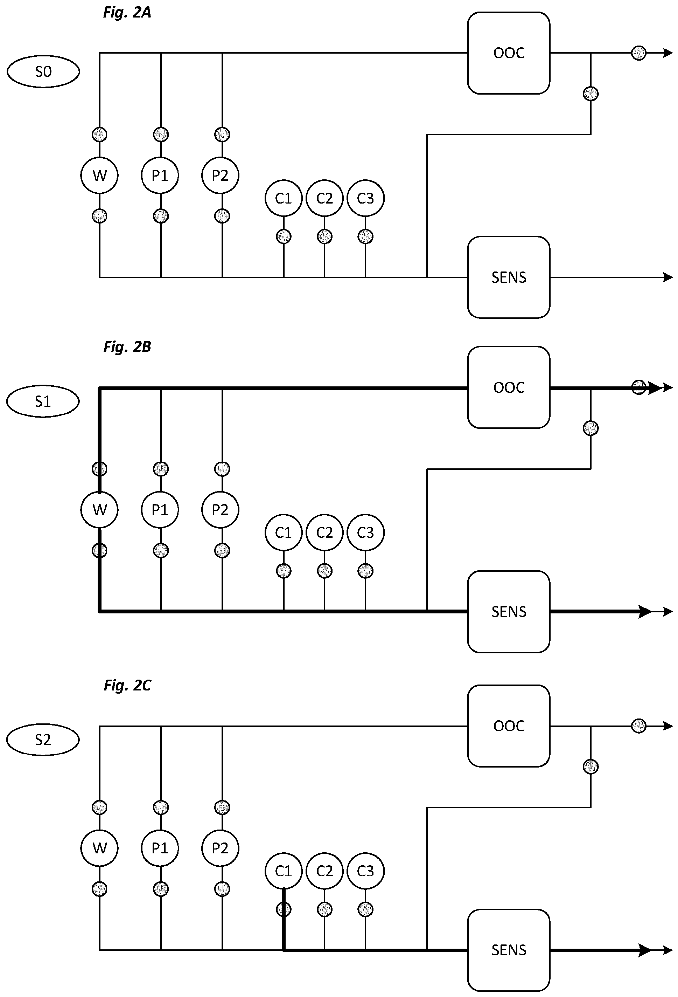

Partant de là, le principe de mise en œuvre de l'invention, à l'aide du système de surveillance décrit ci-dessus, est présenté en liaison avec les figures 2A à 2J. Dans ce processus, il faut comprendre que la commande faite par l'unité de commande UC consiste à contrôler les vannes et les moyens de pompage de manière adaptée pour assurer le passage des liquides dans le circuit selon l'étape considérée.

Figure 2A - S0 : Etat initial dans lequel toutes les vannes sont fermées.

Figure 2B - S1 : L'unité de commande UC contrôle le circuit microfluidique de lavage C_W de sorte que la solution de lavage est injectée dans le composant microfluidique OOC et dans le capteur SENS. Cette étape est optionnelle.

Figure 2C - S2 : L'unité de commande UC contrôle le circuit microfluidique de calibration C_C pour injecter la première solution de calibration C1 à travers le capteur SENS. On peut ainsi faire un étalonnage du capteur électrochimique SENS en déterminant son décalage.

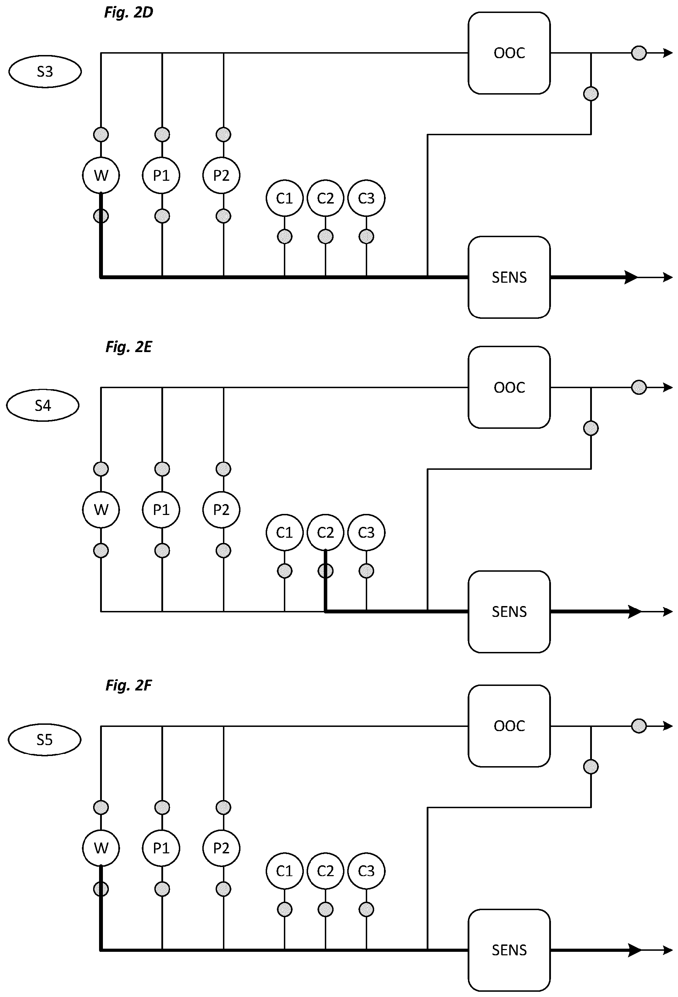

Figure 2D - S3 : L'unité de commande UC contrôle le circuit microfluidique de lavage C_W pour injecter la solution de lavage W dans le capteur SENS, et effacer les traces de la première solution de calibration C1 dans le circuit microfluidique de calibration C_C.

Cette étape est optionnelle.

Figure 2E - S4 : L'unité de commande UC contrôle le circuit microfluidique de calibration C_C pour injecter la deuxième solution de calibration C2 à travers le capteur SENS. On peut ainsi faire une calibration automatique du capteur électrochimique SENS en déterminant à la fois sa sensibilité et son décalage.

Figure 2F - S5 : L'unité de commande UC contrôle le circuit microfluidique de lavage C_W pour injecter la solution de lavage W dans le capteur SENS, et effacer les traces de la deuxième solution de calibration C2 dans le circuit microfluidique de calibration C_C. Cette étape est optionnelle.

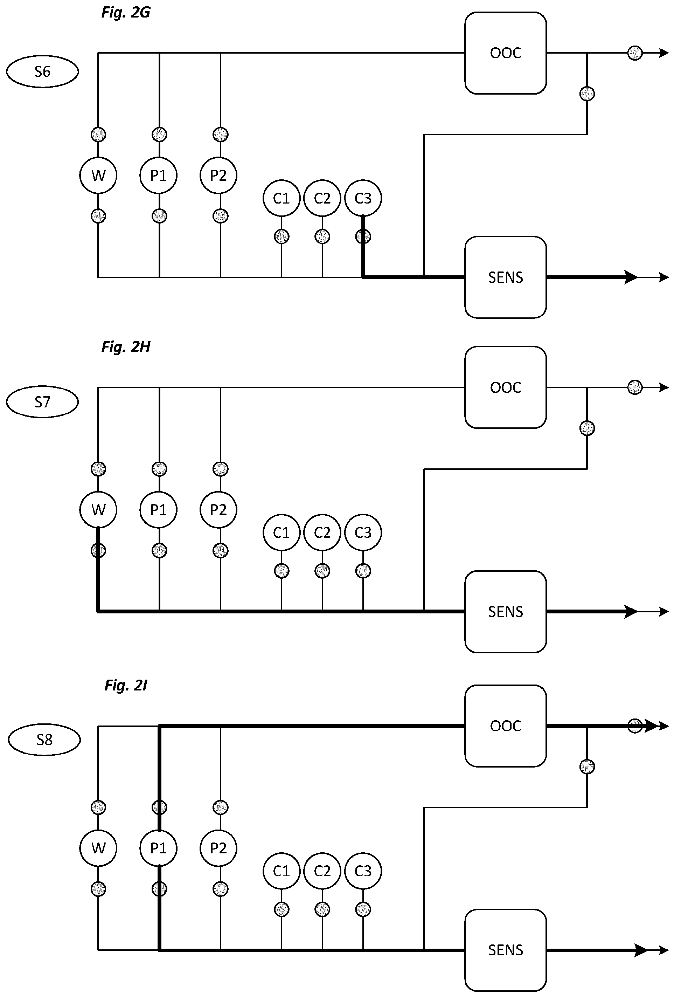

Figure 2G - S6 : L'unité de commande contrôle le circuit microfluidique de calibration C_C pour injecter la troisième solution de calibration C3 à travers le capteur électrochimique SENS. On vient ainsi faire une calibration automatique du capteur SENS en déterminant à la fois sa sensibilité et son décalage. Cette étape reste optionnelle mais avantageuse, puisqu'elle permet d'utiliser trois points de mesure et donc de rendre le procédé plus robuste.

Figure 2H - S7 : L'unité de commande UC contrôle le circuit microfluidique de lavage C_W pour injecter la solution de lavage W dans le capteur SENS, et effacer les traces de la troisième solution de calibration C3 dans le circuit de calibration. Cette étape est optionnelle.

Figure 2I - S8 : L'unité de commande UC contrôle le circuit microfluidique de perfusion C_P pour injecter la première solution de perfusion P1 à la fois dans le composant microfluidique OOC et dans le capteur SENS. De cette manière, le capteur SENS peut déterminer un état de référence de la première solution de perfusion P1.

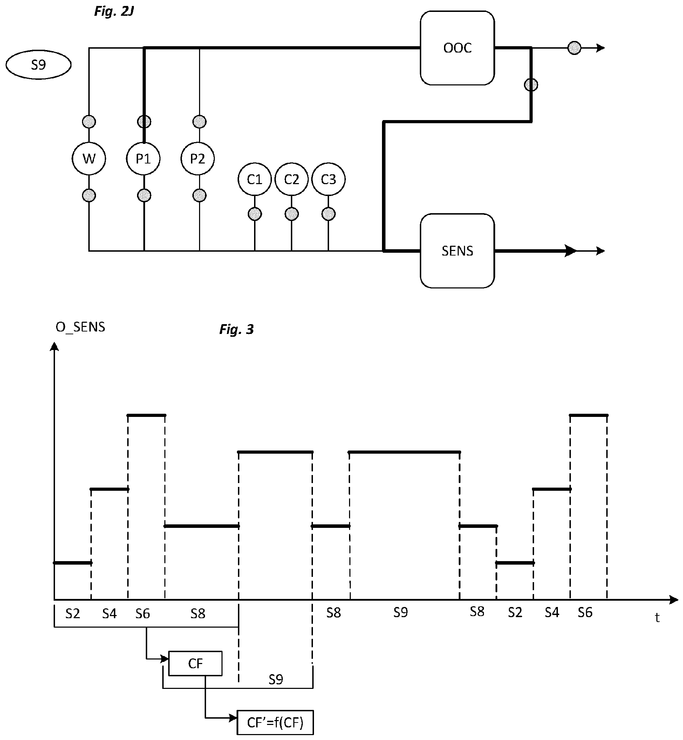

Figure 2J - S9 : L'unité de commande UC contrôle le circuit microfluidique de perfusion C_P pour injecter la première solution de perfusion P1 à travers le composant microfluidique OOC, via la première entrée microfluidique IN1, la première solution de perfusion prenant un deuxième état après passage à travers le composant microfluidique (et donc à travers l'objet biologique). Puis cette première solution de perfusion P1 dans son deuxième état est récupérée au niveau de la première sortie microfluidique OUT1 pour être réinjectée dans le capteur électrochimique SENS sur la deuxième entrée microfluidique IN2, via la liaison spécifique L1 reliant la première sortie microfluidique OUT1 à la deuxième entrée microfluidique IN2. Le capteur SENS est ainsi capable d'effectuer une mesure différentielle, en comparant l'état de référence (S8) de la solution de perfusion P1 et le deuxième état de la solution de perfusion (S9), après passage dans le composant microfluidique. On vient ainsi déterminer l'influence de l'objet biologique sur la solution de perfusion P1. Il peut s'agir de modifications liées à la consommation d'oxygène, d'acidification de la solution, de la présence de sécrétions...

Il est possible de réaliser plusieurs fois les étapes S8 et S9 pour constater les modifications éventuelles dans la solution de perfusion P1, entre son état de référence, et son deuxième état, après passage dans le composant microfluidique. Bien entendu, cette surveillance de la solution de perfusion s'avère d'autant plus pertinente que le capteur SENS est parfaitement calibré.

Il est possible de procéder de manière identique avec la solution de perfusion P2.

En passant régulièrement d'un mode de mesure différentielle à un mode de surveillance (sans passer par la mesure d'un état de référence), il est possible de surveiller si la solution de perfusion conserve ses propriétés au cours du temps, tout en permettant un étalonnage/calibration automatique du capteur.

L'invention permet une calibration régulière du capteur SENS et donc de s'assurer que toute dérive dans les mesures ne provient pas du capteur. En effet, si on constate que les mesures effectuées sur la solution de perfusion dérivent au cours du temps, il serait possible de penser qu'il s'agit d'une dérive du capteur. Mais celui-ci étant calibrée régulièrement, on est sûr qu'il s'agit d'un réel changement au niveau de la solution de perfusion.

Il est ensuite possible de faire une nouvelle calibration du capteur, comme décrit ci-dessus aux étapes S2, S4 et S6. L'unité de commande UC est ainsi capable de suivre l'évolution de la sensibilité du capteur SENS.

La figure 3 montre un diagramme temporel illustrant également le principe de l'invention. Ce diagramme montre la réponse électrochimique du capteur pour chacune des étapes décrites ci-dessus. Selon cette réponse, il est ainsi possible de déterminer la sensibilité et le décalage du capteur et de suivre l'évolution de la concentration d'un analyte cible. Cette concentration est notée C. Le temps est en abscisse et la réponse électrochimique O_SENS du capteur est en ordonnée.

Selon ce diagramme :

S2 + S4 + S6 : il s'agit des trois étapes de calibration, mises en œuvre avec les trois solutions de calibration C1, C2 puis C3. Celles-ci sont injectées dans le capteur uniquement. Il est possible de déterminer la sensibilité α et le décalage β du capteur.

S8 : La première solution de perfusion est injectée à la fois dans le composant microfluidique et dans le capteur. Il est possible de déterminer la concentration CF (solution de perfusion dans son état de référence).

S9 : La première solution de perfusion est injectée dans le composant microfluidique, puis injectée, après passage à travers le composant microfluidique où est situé l'objet biologique, dans le capteur SENS. Il est alors possible de déterminer la concentration CF' (en sortie du composant microfluidique) et de comparer les deux concentrations CF et CF' en faisant une mesure différentielle.

S8 : Cette étape est réalisée à nouveau pour en déduire la concentration CF. Il est ainsi possible de suivre l'évolution de CF au cours du temps.

Il est ensuite possible de répéter une ou plusieurs fois l'enchaînement S9 et S8, pour continuer à suivre l'évolution de CF au cours du temps.

S2+S4+S6 : Il est possible de reprendre une calibration du capteur SENS en vue d'évaluer l'évolution de sa sensibilité au cours du temps. Une diminution de cette sensibilité peut être constatée.

L'invention présente ainsi de nombreux avantages, parmi lesquels :

- Elle permet d'effectuer un étalonnage (décalage) et une calibration (décalage + sensibilité) du capteur à des moments voulus (de manière répétitive durant une expérimentation longue) ;
- Elle permet d'effectuer des mesures différentielles afin d'évaluer un effet induit par l'objet biologique O présent dans le composant, en mesurant l'état de la solution de perfusion avant et après passage par le composant microfluidique contenant l'objet biologique ;
- Elle intègre un circuit microfluidique de calibration (à au moins deux solutions de calibration) ;
- Elle permet la surveillance de la solution de perfusion et permet de vérifier si celle-ci reste stable dans le temps, sans intervention d'un opérateur ;
- Elle permet l'utilisation de la solution de perfusion comme solution d'étalonnage du capteur (un point de mesure), en considérant que la solution de perfusion ne dérive pas elle-même ;
- Comme l'invention repose notamment sur la permutation entre perfusion et calibration, via les différents types de solution, il est avantageux de limiter les volumes morts. L'emploi d'une carte microfluidique permet de remplir cet objectif.

## Revendications

1. Procédé de surveillance d'un objet biologique (O) placé dans un composant microfluidique (OOC), ledit composant microfluidique (OOC) comportant une première entrée microfluidique (IN_1) et une première sortie microfluidique (OUT_1), mis en œuvre à l'aide d'un système de surveillance qui comporte un capteur (SENS), ledit capteur (SENS) comportant une deuxième entrée microfluidique (IN_2) et une deuxième sortie microfluidique (OUT_2), **caractérisé en ce que** le procédé de surveillance comporte :

   - Une première étape d'injection d'une première solution de calibration (C1), via un circuit microfluidique de calibration (C_C), à travers le capteur (SENS), par la deuxième entrée microfluidique (IN_2),
   - Une deuxième étape d'injection d'une deuxième solution de calibration (C2), via le circuit microfluidique de calibration (C_C), à travers le capteur (SENS), par la deuxième entrée microfluidique (IN_2),
   - Une première étape de calibration du capteur (SENS) après ladite première étape d'injection et ladite deuxième étape d'injection,
   - Une première étape de perfusion de l'objet biologique (O) par injection dans un circuit microfluidique de perfusion (C_P) d'une première solution de perfusion (P1) dans le composant microfluidique (OOC), via la première entrée microfluidique (IN_1), ladite première solution de perfusion (P1) étant injectée également dans un premier état dit de référence, en parallèle dans le capteur électrochimique (SENS) via la deuxième entrée microfluidique (IN_2) pour générer à l'aide du capteur des données de mesure de référence,
   - Une deuxième étape de perfusion de l'objet biologique par injection dans le circuit microfluidique de perfusion (C_P) de la première solution de perfusion (P1) dans le composant microfluidique (OOC), via la première entrée microfluidique (IN_1), ladite première solution de perfusion (P1) prenant un deuxième état après avoir traversé l'objet biologique (O),
   - Ladite première solution de perfusion (P1) dans son deuxième état étant injectée dans ledit capteur (SENS) via une liaison fluidique (L1) reliant la première sortie microfluidique (OUT_1) à la deuxième entrée microfluidique (IN_2), pour générer à l'aide du capteur des deuxièmes données de mesure,
   - Une étape de mesure différentielle par le capteur (SENS), par comparaison des données de mesure de référence avec les deuxièmes données de mesure.

**2.** Procédé selon la revendication 1, **caractérisé en ce qu'**il comporte une première étape de lavage du circuit microfluidique de calibration, mise en œuvre après la première étape de calibration.

**3.** Procédé selon la revendication 1 ou 2, **caractérisé en ce qu'**il consiste à répéter une ou plusieurs fois la première étape de perfusion en vue de surveiller la première solution de perfusion (P1).

**4.** Procédé selon la revendication 3, **caractérisé en ce qu'**il consiste à faire suivre chaque itération de la première étape de perfusion par une itération de la deuxième étape de perfusion, en vue de mettre en œuvre une nouvelle étape de mesure différentielle et de suivre l'évolution de la mesure différentielle au cours du temps.

**5.** Système de surveillance d'un objet biologique (O) placé dans un composant microfluidique (OOC), ledit composant microfluidique (OOC) comportant une première entrée microfluidique (IN_1) et une première sortie microfluidique (OUT_1) et étant placé dans un système microfluidique, le système microfluidique comportant également :

- Un capteur (SENS) ayant une deuxième entrée microfluidique (IN_2) et une deuxième sortie microfluidique (OUT_2),
- Un circuit microfluidique de calibration (C_C) connecté sur la deuxième entrée microfluidique (IN_2) du capteur électrochimique (SENS), ledit circuit microfluidique de calibration (C_C) comportant au moins un premier réservoir d'une première solution de calibration (C1) et un deuxième réservoir d'une deuxième solution de calibration (C2),
- Un circuit microfluidique de perfusion (C_P) connecté sur la première entrée microfluidique (IN_1) du composant microfluidique (OOC) et sur la deuxième entrée microfluidique (IN_2) du capteur (SENS), le circuit microfluidique de perfusion (C_P) comportant au moins un réservoir d'une première solution de perfusion (P1),
- Une unité de commande (UC) configurée pour contrôler le circuit microfluidique de perfusion (C_P) et le circuit microfluidique de calibration (C_C),
- **Caractérisé en ce que** :
- Ladite première sortie microfluidique (OUT_1) du composant microfluidique (OOC) est reliée par une liaison microfluidique (L1) à ladite deuxième entrée microfluidique (IN_2) du capteur (SENS),
- L'unité de commande (UC) est configurée pour contrôler le circuit microfluidique de perfusion (C_P) et le circuit microfluidique de calibration microfluidique (C_C) en vue de mettre en œuvre les étapes du procédé de surveillance de l'objet biologique tel que défini dans l'une des revendications 1 à 4.

**6.** Système selon la revendication 5, **caractérisé en ce qu'**il comporte un circuit microfluidique de lavage (C_W) comportant un réservoir d'une solution de lavage (W), connectée à la fois à la première entrée microfluidique (IN_1) du composant microfluidique et à la deuxième entrée microfluidique (IN_2) du capteur.

**7.** Système selon la revendication 5 ou 6, **caractérisé en ce que** le circuit microfluidique de calibration (C_C) comporte un troisième réservoir d'une troisième solution de calibration (C3).

**8.** Système selon l'une des revendications 5 à 7, **caractérisé en ce que** le capteur (SENS) est de type électrochimique.

**Fig. 1**

**Fig. 2A**

**Fig. 2B**

**Fig. 2C**

Fig. 2D

Fig. 2E

Fig. 2F

Fig. 2G

S6

OOC

W    P1    P2

C1    C2    C3

SENS

Fig. 2H

S7

OOC

W    P1    P2

C1    C2    C3

SENS

Fig. 2I

S8

OOC

W    P1    P2

C1    C2    C3

SENS

Fig. 2J

O_SENS

Fig. 3

S2   S4   S6   S8        S8      S9       S8   S2   S4   S6         t

CF

S9

CF'=f(CF)

| | Europäisches Patentamt |
|---|---|
| | European Patent Office |
| | Office européen des brevets |

## RAPPORT DE RECHERCHE EUROPEENNE

Numéro de la demande

EP 25 18 4390

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WO 2021/054280 A1 (TERUMO CORP [JP]) 25 mars 2021 (2021-03-25) * alinéas [0053], [0068], [0069]; revendication 1 * ----- | 1-8 | INV. C12M3/06 B01L3/00 C12M1/34 |
| A | WO 2013/086486 A1 (HARVARD COLLEGE [US]; UNIV VANDERBILT [US]) 13 juin 2013 (2013-06-13) * revendications 1,6,8 * ----- | 1-8 | |
| A | US 9 034 572 B2 (HAMILTON BONADUZ AG [CH]) 19 mai 2015 (2015-05-19) * revendications 1,2 * ----- | 1-8 | |

**DOMAINES TECHNIQUES RECHERCHES (IPC)**

C12M
B01L

Le présent rapport a été établi pour toutes les revendications

| Lieu de la recherche | Date d'achèvement de la recherche | Examinateur |
|---|---|---|
| Berlin | 29 octobre 2025 | Jones, Laura |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 25 18 4390

La présente annexe indique les membres de la famille de brevets relatifs aux documents  brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-10-2025

| Document brevet cité au rapport de recherche | Date de publication | Membre(s) de la famille de brevet(s) | Date de publication |
|---|---|---|---|
| WO 2021054280 A1 | 25-03-2021 | AUCUN | |
| WO 2013086486 A1 | 13-06-2013 | US 2015004077 A1 | 01-01-2015 |
| | | US 2016145554 A1 | 26-05-2016 |
| | | US 2019106665 A1 | 11-04-2019 |
| | | US 2021388301 A1 | 16-12-2021 |
| | | US 2023348831 A1 | 02-11-2023 |
| | | US 2024425788 A1 | 26-12-2024 |
| | | WO 2013086486 A1 | 13-06-2013 |
| US 9034572 B2 | 19-05-2015 | DE 102010001779 A1 | 11-08-2011 |
| | | EP 2363704 A1 | 07-09-2011 |
| | | US 2011236962 A1 | 29-09-2011 |
| | | US 2013288359 A1 | 31-10-2013 |
| | | US 2013291618 A1 | 07-11-2013 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013086486 A1
- WO 2021054280 A1

- US 9034572 B